# EUROPÄISCHE PATENTSCHRIFT

(11) **EP 0 383 982 B1**
(45) Veröffentlichungstag und Bekanntmachung des Hinweises auf die Patenterteilung: **23.06.1993**
(21) Anmeldenummer: 89103174.2
(22) Anmeldetag: 23.02.1989
(51) Int. Cl.: A61B 17/39

(54) **Dreiteilige neutrale Elektrode für ein Hochfrequenz-Chirurgiegerät**
Triple indifferent electrode for a HF surgical apparatus
Electrode indifférente à 3 sections pour un appareil de chirurgie à haute fréquence

(43) Veröffentlichungstag der Anmeldung: 29.08.1990
(73) Patentinhaber: SIEMENS AKTIENGESELLSCHAFT, 80333 München (DE)
(72) Erfinder: Hagen, Uwe, Dipl.-Ing. (FH), D-8550 Forchheim (DE); Feucht, Peter, Dipl.-Ing., D-1000 Berlin 62 (DE)

(56) Entgegenhaltungen:
- DE-A- 3 718 585

## Beschreibung

Die Erfindung betrifft eine dreiteilige neutrale Elektrode für ein Hochfrequenz-Chirurgiegerät, bei der die Teilelektroden nebeneinander und durch isolierende Abstände getrennt auf einem isolierenden elastischen Träger angeordnet sind, wobei die Teilelektroden einander zugewandte, schräg zu einer der Außenkanten des Trägers verlaufende Kanten aufweisen und ein elektrischer Leitungsanschluß für die Teilelektroden an der Seite des Trägers vorgesehen ist, der alle Teilelektroden mit einem Elektrodenabschnitt zugewandt sind, wobei die beiden äußeren Teilelektroden im wesentlichen dreieckförmige Auflageflächen bilden.

Eine neutrale Elektrode dieser Art, bei der die Teilelektroden in einer vorgegebenen Richtung neben- oder übereinander angeordnet und wobei alle drei Teilelektroden im wesentlichen dreieckig ausgebildet sind, ist Gegenstand der DE-A-37 18 585. Zur Bildung einer rechteckförmigen oder quadratischen Gesamtelektrode sind die äußeren dreieckförmigen Teilelektroden um eine Mittelachse spiegelbildlich so angeordnet, daß zwischen die im spitzen Winkel auseinander verlaufenden Hypotenusen der äußeren Dreiecke eine dreieckförmige mittlere Teilelektrode einsetzbar ist, derart, daß die Grundlinie dieses Dreiecks in der Verbindungslinie zwischen den Spitzen der beiden äußeren Dreiecke verläuft.

Aus der US-PS 4,770,173 ist ferner eine dreiteilige neutrale Elektrode für die HF-Chirurgie bekannt, bei der zwei Teilelektroden trapezförmig ausgebildet sind und einander zugewandte, schräg zu einer vorgegebenen Richtung verlaufende Kanten aufweisen. In der vorgegebenen Richtung ist nach den beiden trapezförmigen Teilelektroden eine weitere, im wesentlichen rechteckig ausgebildete Teilelektrode vorgesehen. Zwischen den Teilelektroden befinden sich Isolierstreifen.

In der DE-A-35 44 443 ist eine Schaltungsanordnung zur Überwachung der neutralen Elektrode eines HF-Chirurgiegeräts auf flächiges Anliegen der Elektrode am Körper des Patienten beschrieben. Durch Fehlapplikation oder Ablösen der neutralen Rückstrom-Elektrode kann es in der HF-Chirurgie zu unerwünschten Verbrennungen des Patienten kommen. Durch mehrteilige neutrale Elektroden und eine Überwachungseinrichtung soll das Ablösen der Elektrode rechtzeitig erkannt und dem Arzt signalisiert werden. Es ist bereits bekannt, zur Erhöhung der Patientensicherheit die neutrale Elektrode in mehrere Teilelektroden zu unterteilen. Bei einer solchen Einrichtung wird ein Hilfs- oder Meßstrom von einem Meßstromgeber zur einen Teilelektrode, von dort über den Patienten zur anderen Teilelektrode und von dieser schließlich zurück zum Meßstromgeber geleitet und überwacht. Wenn dieser Stromkreis geschlossen ist, dann ist sichergestellt, daß jede Teilelektrode praktisch ganzflächig am Patienten anliegt, so daß der eigentliche HF-Arbeitsstrom appliziert werden kann. Eine solche unterteilte neutrale Elektrode ermöglicht somit die Feststellung, ob diese richtig angebracht ist.

Für diese bekannten mehrteiligen Elektroden ist jedoch eine Anordnung der Teilelektroden gewählt, die ein teilweises Ablösen von Teilelektroden ermöglicht, ohne daß dadurch die Überwachungsschaltung anspricht und Ablösealarm auslöst. Dieser Alarm erfolgt aufgrund der bekannten Elektrodenausbildung oftmals zu spät, da die Überwachungseinrichtung auf das Ablösen der Teilelektroden je nach Ablöserichtung der Elektrode unterschiedlich anspricht. Die bekannten mehrteiligen Elektroden sind so angeordnet bzw. ausgebildet, daß sie nach einer Ablöse-Symmetrie-Linie vom Körper des Patienten teilweise abgezogen werden können, ohne daß die beispielsweise Impedanzmessungen zwischen den auf dem Körper des Patienten aufliegenden Teilflächen der mehrteiligen Elektrode ausführende Überwachungseinrichtung rechtzeitig anspricht und Alarm auslöst.

Wegen der von Patient zu Patient unterschiedlichen Leitwerte des Patientengewebes liefert eine Widerstands- oder Kapazitätsmessung zwischen zwei Teilelektroden einer neutralen HF-Chirurgie-Elektrode noch kein ausreichend sicheres Ergebnis dafür, ob die Kontaktfläche zwischen der Elektrode und dem Patienten ausreichend ist, um Verbrennungen des Patienten zu vermeiden. Weist beispielsweise das Gewebe eines Patienten nur ein Drittel des Gewebewiderstandes eines anderen Patienten auf, so kann bei diesem niedrigen Gewebewiderstand etwa ein Drittel der Elektrodenfläche ausreichend sein, um einen voreingestellten Grenzwert für die Messung einzuhalten. Aus diesem Grunde ist es sinnvoll, mit Elektroden zu arbeiten, die mehrere Teilflächen aufweisen und bei denen durch eine Symmetriemessung von Teilströmen auf den gleichmäßigen Kontakt mit dem Patienten geschlossen wird. Für die Symmetriemessung kann dabei ein Hilfsstrom oder der HF-Chirurgiestrom selbst verwendet werden.

Der Erfindung liegt die Aufgabe zugrunde, eine dreiteilige neutrale Elektrode zu schaffen, die diesen Nachteil vermeidet, einfach herstellbar ist und ein gutes Anliegen am Patienten gewährleistet.

Diese Aufgabe wird erfindungsgemäß dadurch gelöst, daß die langen Kanten der äußeren Teilelektroden mit Abstand und parallel verlaufen und daß eine mittlere streifenförmige Teilelektrode zwischen den langen Kanten der äußeren Teilelektroden angeordnet ist.

Die dreiteilige neutrale Elektrode nach der Erfindung besitzt keine Ablöse-Symmetrie-Linie, nach der ein weitgehendes Ablösen der Elektrode vom Patientenkörper möglich wäre, ohne daß aufgrund von Überwachungsmessungen (z.B. Impedanzmessungen) zwischen den Teilelektroden - wegen sich kaum verändern der Meßwerte - die Überwachungsschaltung Alarm auslöst. Für eine Impedanzüberwachung zwischen den Teilflächen der dreiteiligen Elektrode kann als Einspeiseelektrode für den zur Messung benötigten Hilfsstrom die mittlere oder eine der äußeren Teilelektroden gewählt werden.

Die Erfindung schlägt eine dreiteilige Elektrodenform vor, die so gestaltet ist, daß beim unbewußten oder unbeabsichtigten Ablösen der Teilelektrodenflächen vom Patienten kaum eine Symmetrie der Restflächen eingehalten werden kann, insbesondere wenn davon ausgegangen wird, daß die Linie der Ablösung eine Gerade ist und weiterhin die Gerade parallel zu einer der Außenkanten der neutralen Elektrode liegt.

Bei der neutralen Elektrode nach der Erfindung sind zwar Ablösegeraden denkbar, bei denen die annähernde Symmetrie der Restflächen erhalten bleibt, jedoch verlaufen diese kritischen Ablösegeraden schräg zu den Außenkanten der Elektrode und weiterhin verändern die kritischen Ablösegeraden mit zunehmender Ablösung ständig ihren Winkel zu den Außenkanten der Elektrode.

Die erfindungsgemäße Elektrodenform ist auch für den Anwendungsfall geeignet, daß nur das Stromverhältnis zwischen den beiden dreieckförmigen äußeren Teilelektroden ausgewertet wird, wobei die mittlere Teilelektrode z.B. zur Einspeisung eines Hilfsstromes dient.

Vorteilhafte Ausgestaltungen der dreiteiligen neutralen Elektrode sind in den Patentansprüchen 2 bis 8 gekennzeichnet.

Der Leitungsanschluß der dreiteiligen neutralen Elektrode ist so ausgebildet, daß die Elektrode nur in Verbindung mit einem dazupassenden Stecker des Geräte-Elektrodenanschlußkabels kuppelbar ist. Damit ist sichergestellt, daß die richtige Elektrodenart an das HF-Chirurgiegerät angeschlossen wird. Der Anschluß von falschem Zubehör ist dadurch ausgeschlossen.

Im folgenden werden Ausführungsformen der Erfindung anhand von vier Figuren erläutert. Es zeigen:
Figur 1 eine dreiteilige neutrale Elektrode für ein HF-Chirurgiegerät in Draufsicht, mit in x-Richtung nebeneinander angeordneten Teilelektroden;
Figur 2 eine dreiteilige neutrale Elektrode, die im Vergleich zu Figur 1 im wesentlichen eine rechteckige Form hat und deren Teilelektroden in x-Richtung übereinander angeordnet sind;
Figur 3 eine weitere dreiteilige Elektrode mit einer streifenförmigen fünfeckigen mittleren Teilelektrode;
Figur 4 eine dreiteilige Elektrode, deren streifenförmige, viereckige mittlere Teilelektrode zwischen zwei Teilelektroden liegt, die die Form eines gleichschenkeligen Dreiecks aufweisen, wobei die Ecken der Teilelektroden gerundet ausgeführt sind.

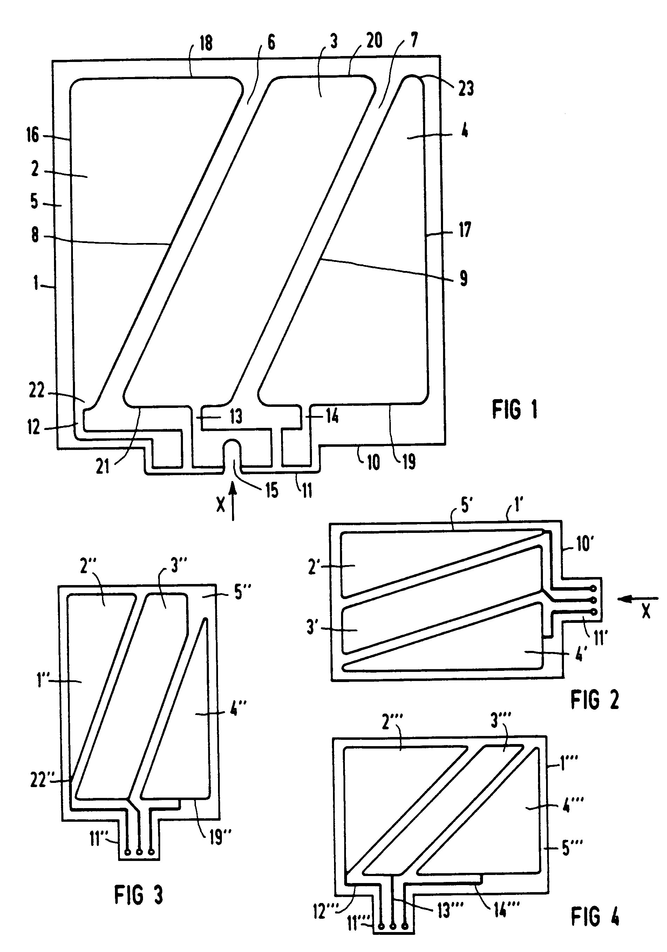

Nach Figur 1 umfaßt die neutrale Elektrode 1 für ein HF-Chirurgiegerät drei flächenhafte Teilelektroden 2, 3 und 4, die auf einem elastischen Träger 5 so angeordnet sind, daß die Teilelektroden durch Isolierstreifen 6, 7 oder Streifen geringer elektrischer Leitfähigkeit voneinander getrennt sind. Die Teilelektroden 2, 3, 4 sind in einer vorgegebenen Richtung x, z.B. in der Anlegerichtung der Elektrode, nebeneinander angeordnet. Die beiden äußeren Teilelektroden 2 und 4 sind im wesentlichen dreieckig ausgebildet und zwischen ihren Hypotenusen 8 bzw. 9, die schräg zur Richtung x sowie mit Abstand parallel verlaufen, befindet sich die mittlere, streifenförmig ausgebildete dritte Teilelektrode 3. Die Teilelektroden bestehen jeweils aus einer elektrisch leitenden Folie, z.B. einer Metallfolie, oder einem Metallnetz und sind auf dem biegsamen Träger 5 befestigt. Der Träger 5, der bevorzugt selbstklebend ausgeführt ist und aus einem Gummi oder Kunststoff bestehen kann, ist beim Ausführungsbeispiel nach Figur 1 im wesentlichen quadratisch ausgebildet. Der Träger 5 steht am Rande über die drei Teilelektroden 2, 3 und 4 über. An derjenigen Seite 10 des Trägers 5, der alle Teilelektroden 2 bis 4 mit einem Elektrodenabschnitt zugewandt sind, weist die Elektrode einen Leitungsanschluß 11 auf. Dieser Leitungsanschluß 11 nimmt drei Verbindungsleitungen 12, 13 und 14 für die Teilelektroden 2, 3 bzw. 4 auf.

Der Leitungsanschluß 11 ist so bemessen und geformt, daß nur eine Kupplung mit einem zugehörigen, nicht gezeichneten Stecker eines Elektrodenanschlußkabels eines HF-Chirurgiegerätes möglich ist. Der Anschluß beliebiger neutraler Elektroden an das HF-Chirurgiegerät wird dadurch vermieden, d.h. der Anschluß von falschem Zubehör ist nicht möglich. Als mechanische Kodierung 15 weist der Leitungsanschluß 11 beispielsweise eine Kerbe von bestimmter Form und Anordnung auf.

Die Anordnung und Form der Teilelektroden ist gemäß Figur 1 so gewählt, daß die langen Katheten 16 bzw. 17 der äußeren Teilelektroden 2 bzw. 4 im wesentlichen die Länge der Elektrode 1 in der x-Richtung bestimmen. Die kurzen Katheten 18 bzw. 19 der äußeren Teilelektroden fluchten mit Stirnkanten 20 bzw. 21 der mittleren Teilelektrode 3. Auf gleicher Bezugslinie liegen die Spitzen 22 bzw. 23 der äußeren Teilelektroden 2 bzw. 4.

In Figur 2 ist eine rechteckförmige Ausführung der Elektrode 1' gezeigt, die an einer der schmalen Stirnseiten 10' des Trägers 5' den Leitungsanschluß 11' aufweist. Die Teilelektroden 2', 3' und 4' sind in x-Richtung übereinander angeordnet.

Die Ausführung nach Figur 3 stellt ebenfalls eine rechteckförmige Elektrode 1'' dar, deren äußere Teilelektroden 2'' und 4'' rechtwinkelige Dreiecke bilden. Die streifenförmige mittlere Teilelektrode 3'' ist beispielsweise mit einer geraden und einer abgewinkelten Stirnkante fünfeckig geformt.

Bei der in Figur 4 dargestellten Ausführungsform sind die beiden Teilelektroden 2''' und 4''' als gleichschenkelige rechtwinkelige Dreiecke ausgebildet. Damit die Verbindungsleitungen 12''', 13''' und 14''' zwischen den Teilelektroden und dem Leitungsanschluß 11''' etwa gleich lang ausgelegt werden können, ist der Leitungsanschluß 11''' außermittig am Träger 5''' der Elektrode 1''' angebracht.

Die mehrteilige neutrale Elektrode nach der Erfindung zeichnet sich dadurch aus, daß der leitende Flächenquerschnitt der Elektrode senkrecht zur Richtung x über die Länge der Elektrode etwa konstant bleibt. Dadurch wird die Kapazitätsmessung für die Überwachung der Elektrode, ob diese richtig am Körper des Patienten angebracht ist, verbessert. Die Kapazitätsmeßeinrichtung enthält auch eine Einrichtung zur Feststellung, ob die gemessene Kontaktfläche zwischen der neutralen Elektrode und dem Patienten einen vorbestimmten Wert übersteigt, der einem sicheren Betrieb des HF-Chirurgiegerätes entspricht. Anstelle einer Kapazitätsmessung kann auch eine Schaltungsanordnung gemäß der DE-A-35 44 443 vorgesehen werden. Danach wird eine Messung der HF-Teilströme der mehrteiligen neutralen Elektrode durchgeführt und ein Alarmsignal aus einer Verknüpfung, insbesondere einer Verhältnisbildung, der Teilströme gebildet. Wird aufgrund der Verknüpfung der Meßsignale eine annähernd gleichmäßige Stromverteilung auf die Teilelektroden festgestellt, dann dürften Verbrennungen des Patienten ausgeschlossen sein und das Alarmsignal wird nicht ausgelöst. Alarm und Sicherheitsmaßnahmen werden dagegen eingeleitet, wenn die Erfassung der Hochfrequenz-Teilströme und deren gerätetechnische Verknüpfung anzeigt, daß einer der Teilströme nennenswert überwiegt.

## Patentansprüche

1. Dreiteilige neutrale Elektrode für ein Hochfrequenz-Chirurgiegerät, bei der die Teilelektroden nebeneinander und durch isolierende Abstände getrennt auf einem isolierenden, elastischen Träger (5) angeordnet sind, wobei die Teilelektroden einander zugewandte, schräg zu einer der Außenkanten des Trägers verlaufende Kanten aufweisen und ein elektrischer Leitungsanschluß (11) für die Teilelektroden an der Seite des Trägers vorgesehen ist, der alle Teilelektroden mit einem Elektrodenabschnitt zugewandt sind, wobei die beiden äußeren Teilelektroden (2, 4) im wesentlichen dreieckförmige Auflageflächen bilden, **dadurch gekennzeichnet,** daß die langen Kanten (8 bzw. 9) der äußeren Teilelektroden mit Abstand und parallel verlaufen und daß eine mittlere, streifenförmige Teilelektrode (3) zwischen den langen Kanten der äußeren Teilelektroden angeordnet ist.

2. Elektrode nach Anspruch 1, **dadurch gekennzeichnet,** daß die äußeren Teilelektroden (2, 4) kongruente, um die streifenförmige mittlere Teilelektrode (3) so angeordnete Dreiecke bilden, daß sich eine rechteckförmige oder quadratische Elektrodenform ergibt.

3. Elektrode nach Anspruch 1, **dadurch gekennzeichnet,** daß die äußeren Teilelektroden (2, 4) als rechtwinkelige Dreiecke und die mittlere Teilelektrode (3) als vier- bis sechseckiger Streifen ausgebildet sind, daß die Katheten der Dreiecke parallel im Bereich der Stirnkanten des rechteckförmigen oder quadratischen Trägers (5) verlaufen und daß die zwischen den Hypotenusen (8, 9) der Dreiecke angeordnete mittlere Teilelektrode (3) mit ihrer dem Leitungsanschluß (11) zugewandten Stirnkante (21) sowie mit der dem Leitungsanschluß gegenüberliegenden Stirnkante (20) parallel zu der einen Kathete (18 bzw. 19) des jeweiligen Dreiecks verläuft.

4. Elektrode nach Anspruch 1, **dadurch gekennzeichnet,** daß die äußeren Teilelektroden (2, 3) spitzwinkelige Dreiecke bilden, wobei ein Dreieck (2) mit seiner Spitze (22) und das andere Dreieck (4) mit einer Kathete (19) dem Leitungsanschluß (11) zugewandt ist und die Stirnseiten (20, 21) der mittleren Teilelektrode (3) parallel zu Katheten (18 bzw. 19) der dreieckförmigen äußeren Teilelektroden verlaufen.

5. Elektrode nach Anspruch 1, **dadurch gekennzeichnet,** daß die äußeren Teilelektroden (2''', 4''') gleichschenkelige rechtwinkelige Dreiecke bilden.

6. Elektrode nach Anspruch 1, mit einem laschenförmig am Träger angeordneten Leitungsanschluß, **dadurch gekennzeichnet,** daß der Leitungsanschluß (11) eine zu einem zugehörigen Stecker eines Elektrodenanschlußkabels passende mechanische Kodierung (15) aufweist.

7. Elektrode nach Anspruch 6, **dadurch gekennzeichnet,** daß der Leitungsanchluß (11) aus einer Anschlußlasche am Träger (5) der Elektrode (1) besteht, die die elektrischen Leitungsverbindungen (12, 13, 14) zu den Teilelektroden (2, 3, 4) und eine bestimmte, zum Stecker passende Laschenform aufweist.

8. Elektrode nach Anspruch 6, **dadurch gekennzeichnet,** daß der Leitungsanschluß (11''') außermittig am Träger (5''') der Elektrode (1''') angebracht ist.

## Claims

1. Three-part neutral electrode for a high-frequency surgical instrument, in which the part-electrodes are arranged side by side, separated by insulating clearances, on an insulating elastic carrier (5), with the part-electrodes having facing edges which extend obliquely relative to one of the outer edges of the carrier and with an electrical line terminal (11) being provided for the part-electrodes on the side of the carrier towards which all the part-electrodes face with one electrode section, with the two outer part-electrodes (2, 4) forming substantially triangular contact surfaces, characterised in that the long edges (8 and 9 respectively) of the outer part-electrodes extend with clearance and in a parallel manner and in that a central strip-shaped part-electrode (3) is arranged between the long edges of the outer part-electrodes.

2. Electrode according to claim 1, characterised in that the outer part-electrodes (2, 4) form congruent triangles which are arranged about the strip-shaped central part-electrode (3) in such a way that a rectangular or square electrode form results.

3. Electrode according to claim 1, characterised in that the outer part-electrodes (2, 4) are formed as right-angled triangles and the central part-electrode (3) is formed as a four- to six-cornered strip, in that the perpendicular sides of the triangles extend in a parallel manner in the region of the front edges of the rectangular or square carrier (5) and in that the central part-electrode (3) arranged between the hypotenuses (8, 9) of the triangles extends so that its front edge (21), which faces the line terminal (11), and also so that the front edge (20), which lies opposite the line terminal, are parallel to the one perpendicular side (18 and 19 respectively) of the respective triangle.

4. Electrode according to claim 1, characterised in that the outer part-electrodes (2, 3) (sic) form acute triangles, with one triangle (2) facing the line terminal (11) with its vertex (22) and the other triangle (4) facing said terminal with a perpendicular side (19) and the end faces (20, 21) of the central part-electrode (3) extending so as to be parallel to perpendicular sides (18 and 19 respectively) of the triangular outer part-electrodes.

5. Electrode according to claim 1, characterised in that the outer part-electrodes (2''', 4''') form isosceles right-angled triangles.

6. Electrode according to claim 1, having a line terminal which is arranged in a lug-shaped manner on the carrier, characterised in that the line terminal (11) has a mechanical coding (15) which fits a pertinent plug of an electrode terminal cable.

7. Electrode according to claim 6, characterised in that the line terminal (11) consists of a terminal lug on the carrier (5) of the electrode (1), which lug has the electrical line connections (12, 13, 14) to the part-electrodes (2, 3, 4) and a certain lug form which fits the plug.

8. Electrode according to claim 6, characterised in that the line terminal (11''') is provided eccentrically on the carrier (5''') of the electrode (1''').

## Revendications

1. Électrode neutre formée de trois éléments pour un appareil chirurgical à haute fréquence, dans laquelle les électrodes partielles sont disposées côte-à-côte en étant séparées par des distances d'isolation, sur un support élastique isolant (5), et dans laquelle les électrodes partielles possèdent des bords tournés les uns vers les autres et disposés obliquement par rapport aux bords extérieurs du support, et il est prévu une borne (11) pour un conducteur électrique pour les électrodes partielles, du côté du support, vers lequel toutes les électrodes partielles sont tournées avec une section d'électrode, les deux électrodes extérieures partielles (2,4) formant des surfaces d'appui sensiblement triangulaires, caractérisée par le fait que les longs bords (8 ou 9) des électrodes partielles extérieures sont distants et parallèles et qu'une électrode partielle médiane en forme de bande (3) est disposée entre les longs bords des électrodes partielles extérieures.

2. Électrode suivant la revendication 1, caractérisée par le fait que les électrodes partielles extérieures (2,4) forment des triangles acutangles, qui sont disposés autour de l'électrode partielle médiane en forme de bande (3) de sorte que l'on obtient une forme d'électrode rectangulaire ou carrée.

3. Électrode suivant la revendication 1, caractérisée par le fait que les électrodes partielles extérieures (2,4) sont réalisées sous la forme de triangles rectangles et que l'électrode partielle médiane (3) est réalisée sous la forme d'une bande ayant de quatre à six côtés, que les côtés de l'angle droit des triangles sont parallèles aux et au voisinage des bords frontaux du support rectangulaire ou carré (5) et que l'électrode partielle médiane (3), disposée entre les hypothénuses (8,9) des triangles, est disposée de telle sorte que son bord frontal (21), tourné vers la borne (11) de raccordement du conducteur, ainsi que le bord frontal (20) situé à l'opposé de la borne de raccordement du conducteur sont parallèles à l'un des côtés (18 ou 19) de l'angle droit du triangle respectif.

4. Électrode suivant la revendication 1, caractérisée par le fait que les électrodes partielles extérieures (2,3) forment des triangles pointus, un triangle (2) étant tourné avec sa pointe (22) et l'autre (4) avec un côté (19) de son angle droit vers la borne (11) de raccordement du conducteur, et les faces frontales (20,21) de l'électrode partielle médiane (3) s'étendant parallèlement aux côtés de l'angle droit (18,19) des électrodes partielles extérieures de forme triangulaire.

5. Électrode suivant la revendication 1, caractérisée par le fait que les électrodes partielles extérieures (2''',4''') forment des triangles rectangles isocèles.

6. Électrode suivant la revendication 1, comportant une borne de raccordement du conducteur, disposée sous la forme d'une patte sur le support, caratérisée par le fait que la borne (11) de raccordement du conducteur possède un codage mécanique (15) adapté à une fiche mâle associée d'un câble de raccordement de l'électrode.

7. Électrode suivant la revendication 6, caractérisé par le fait que la borne (11) de raccordement du conducteur est constituée par une patte de raccordement située sur le support (5) de l'électrode (1), qui possède les connexions électriques (12,13,14) du conducteur avec les électrodes partielles (2,3,4) et une forme de patte déterminée adaptée à la fiche mâle.

8. Électrode suivant la revendication 6, caractérisée par le fait que la borne (11''') de raccordement du conducteur est disposée d'une manière excentrée sur le support (5''') de l'électrode (1''').
